# EUROPEAN PATENT APPLICATION

(11) **EP 2 030 573 A1**
(43) Date of publication of application: **04.03.2009**
(21) Application number: 07767053.7
(22) Date of filing: 06.06.2007
(51) Int. Cl.: A61B 8/08

(54) **ULTRASONOGRAPHIC DEVICE**

(30) Priority: 06.06.2006 JP 2006157055
(71) Applicant: Hitachi Medical Corporation, Tokyo 101-0021 (JP)
(72) Inventor: OSAKA, Takashi, Tokyo 101-0021 (JP)
(74) Representative: Erny, Tobias
(86) International application number: PCT/JP2007/061421
(87) International publication number: WO 2007/142255

(57) **Abstract**

It is possible to acquire the relationship between tomography data as a luminance value of an ultrasonic diagnostic image and elasticity data on a tissue in a target local region.

ROI information calculation means divides an ROI into a plurality of divided regions.

Displacement amount calculation means (21) calculates an inter-frame displacement amount, adds per-region information to a boundary of the divided regions, and outputs the displacement amount data to elasticity calculation means (22).

The elasticity calculation means (22) calculates elasticity data according to the displacement amount.

Elasticity information analysis means (23) calculates a representative value of the elasticity data on the actual tissue and outputs address information of the region other than the actual tissue to signal analysis means (17).

The signal analysis means (17) calculates the representative value of the tomography data on the actual tissue according to the tomography data generated by ultrasonic diagnostic image calculation means (16) and the address information.

The calculated tomography data and the elasticity data are displayed on display means (20) by various display methods.

## Description

### Technical Field

The present invention is related to an ultrasonic diagnostic apparatus for displaying a tomographic image of an object to be examined using ultrasonic waves, and an ultrasonic diagnostic apparatus having a function for calculating and displaying elasticity data such as strain or elasticity modulus, etc. of each point on the tomographic image.

### Background Art

A conventional ultrasonic diagnostic apparatus comprises:
ultrasonic transmitting/receiving means for transmitting/receiving ultrasonic waves to/from an object;
tomographic scanning means for repeatedly acquiring diagnostic image data on the inside of the object including motion tissues in predetermined cycle using ultrasonic echo signals from the ultrasonic transmitting/ receiving means; and
image display means for displaying time-series data acquired by the tomographic scanning means,
for displaying a tomographic image of biological tissues in the body of the object using luminance value data.

On the other hand, recently, the ultrasonic diagnostic apparatus is used for calculating elasticity data such as strain or elasticity modulus of biological tissues and displaying the acquired data as an elastic image. One example of such ultrasonic diagnostic apparatuses calculates elasticity data (hardness) using pulsation of a blood vessel (for example, Patent Document 1).
The reason that such elasticity data is considered useful is because, for example, since tissues get harder due to factors such as lack of tissue flexibility caused by increasing of age or plaque accumulated on a blood vessel wall, elasticity data is considered as acutely reflecting characterization of diseased tissues. Particularly, accumulation of plaque on a blood vessel wall has a risk of breaking down and clogging up peripheral blood vessels, thus acquisition of elasticity data of plaque is considered as providing valuable information to clinical site. Also, elasticity data of plaque is allegedly associated to luminance value of tomographic images obtained by ultrasonic diagnostic apparatus. For example, lipid-based plaque with a greater risk of breaking down has lower luminance value on a tomographic image, and fiber-based plaque with a lower risk of breaking down such as calcified plaque is displayed having higher luminance value on a tomographic image.

Patent Document 1: JP-A-H5-317313

### Disclosure of the Invention

### Problems to be Solved

However, the ultrasonic diagnostic apparatus comprising the conventional elastic image display means has the unsolved problem that, for example, while elasticity data of tissues can be obtained it cannot obtain the relationship between tomography data that is a luminance value of an ultrasonic tomographic image and elasticity data of a target local region.

The objective of the present invention is to provide an ultrasonic diagnostic apparatus capable of acquiring the relationship between tomography data and elasticity data in a target local region.

### Means for Solving the Problem

In order to solve the above-described problem, the ultrasonic diagnostic apparatus of the present invention comprises:
an ultrasonic probe for transmitting/receiving ultrasonic waves to/from an object to be examined;
elasticity data calculation means for calculating elasticity data indicating tissue displacement based on output signals from the ultrasonic probe;
tomography data calculation means for calculating tomography data indicating tissue characterization based on the output signals from the ultrasonic probe; and
display means for displaying the elasticity data and the tomography data,
characterized in further comprising:
target region dividing/setting means for setting a region of interest by dividing it into a plurality of regions in the display means,
wherein the elasticity data calculation means and/or the tomography data calculation means perform calculation for each divided region.

An ultrasonic diagnostic apparatus is for transmitting/receiving ultrasonic waves to/from an object, calculating and displaying elasticity data or tomography data of tissues based on output signals from the ultrasonic probe.

The ultrasonic diagnostic apparatus of the present invention divides a region of interest including the diagnostic target tissues into a plurality of regions, calculates elasticity data or tomography data for each divided region, and displays the elasticity data or tomography data for each divided region.

In this manner, the region of interest can be divided and set down as a plurality of regions for analysis. Also, the result of analysis can be displayed for each divided region.

Also, elasticity data calculation means of the present invention calculates the representative value (for example, average value, etc.) of elasticity data for each divided region, and tomography data calculation means calculates the representative value of tomography data (for example, average value, etc. of tomographic image luminance value) for each divided region.

Also, display means displays distribution of the representative value of the elasticity data and the representative value of the tomogrpahic data for each divided region.

In this manner, the representative value of elasticity data and tomography data can be calculated for each divided region, and a distribution chart indicating the relationship between the representative value of the elasticity data and the representative value of the tomography data can be developed. Also, the ultrasonic diagnostic apparatus of the present invention calculates address information of actual tissues based on displacement amount distribution of elasticity data, and elasticity data calculation means and tomography data calculation means calculate tomography data or elasticity data by eliminating elasticity data and tomography data in the regions other than the actual tissues based on the address information.

Accordingly, elasticity data and tomography data of actual tissues for each divided region can be accurately calculated.

Also, elasticity data calculation means calculates elasticity data by adding region identification data for identifying the boundary of the divided regions to elasticity data (displacement amount data) between the frames.

The ultrasonic diagnostic apparatus is capable of mutually identifying boundary regions of the divided regions by the region identification data in the respective signal processes.

### Effect of the Invention

In this way, in accordance with the present ultrasonic diagnostic apparatus, since a region of interest is divided into a plurality of regions so as to calculate tomography data or elasticity data for each divided region, it is possible to obtain the relationship between tomography data and elasticity data in the target local region and perform ultrasonic diagnosis visually from multifaceted perspectives.

### Brief Description of the Diagrams

Fig. 1 is a block configuration diagram of an ultrasonic diagnostic apparatus related to the present embodiment.
Fig. 2 is a general flow chart of calculation process to be performed by ultrasonic diagnostic apparatus 1.
Fig. 3 shows an example of screens displayed on a display means 20, and a console 25.
Fig. 4 is an enlarged view in the vicinity of ROI 20d displayed on an elastic image screen 20b.
Fig. 5 is a block configuration diagram of a displacement amount calculation means 21.
Fig. 6 is a block configuration diagram of an analyzed region information adding means 21c.
Fig. 7 is a diagram for explaining addition of a region identification data 43.
Fig. 8 is a block configuration diagram of an elasticity data analysis means 23.
Fig. 9 shows a block configuration diagram of an elasticity data analysis means 23a.
Fig. 10 is an explanatory diagram of a processing to be performed by an elasticity data processing means 23a.
Fig. 11 shows a block configuration diagram of an elasticity data processing means 23b.
Fig. 12 is a block configuration diagram of a signal analysis means 17.
Fig. 13 is an explanatory diagram for calculation process of the representative value of tomography data.
Fig. 14 is a display example of a distribution chart of the representative value of tomography data and elasticity data.
Fig. 15 illustrates operation to be performed by a distribution chart creating/comparing means 17f and a display example thereof.
Fig. 16 shows a display example of tomography data distribution.
Fig. 17 shows a display example of tomography data distribution.
Fig. 18 shows a display example of tomography data distribution.
Fig. 19 shows a kind of probe 13 and an example of the ROI setting method.

### Description of the Symbols

1... ultrasonic diagnostic apparatus, 3... object, 11... transmission means, 12... transmission/reception separating means, 13... probe, 14... reception means, 15... phasing addition means, 16... ultrasonic tomographic image calculation means, 17... signal analysis means, 18... black and white signal information converting means, 19... switching/adding means, 20... display means, 21... displacement amount calculation means, 22... elasticity calculation means, 23... elasticity information analysis means, 24... color signal data converting means, 25... console, 26... ROI data calculation means, 20d... ROI, 20g... divided region, 43... region identification data, 51... actual tissue, 55... address information data

### Best Mode for Carrying Out the Invention

Hereinafter, preferable embodiment of the ultrasonic diagnostic apparatus related to the present invention will be described in detail referring to the attached diagrams. In the description and the attached diagrams below, description of components having the same function and configuration will be omitted by appending the same symbols.

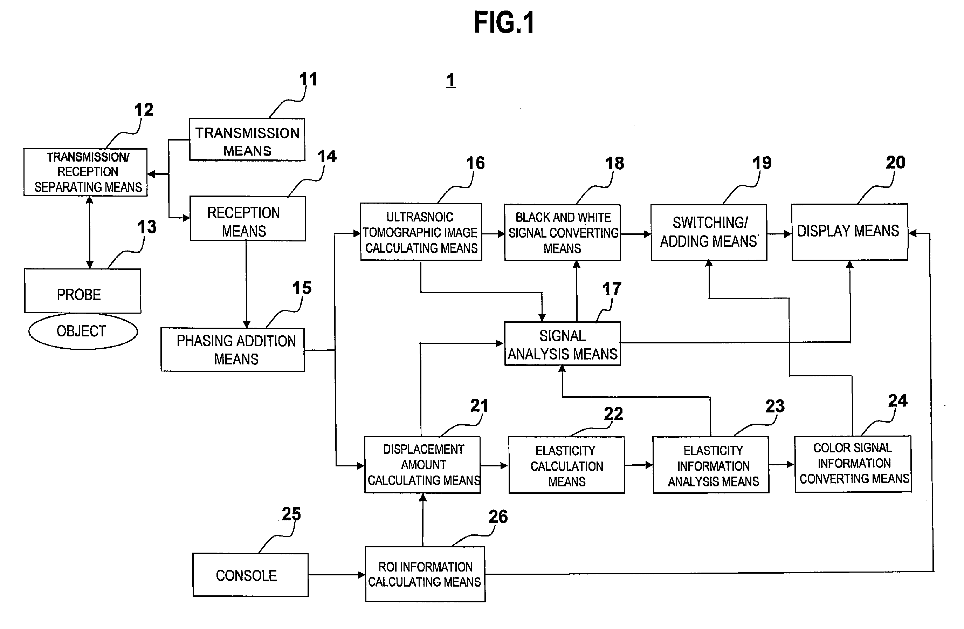

Fig. 1 is a block configuration diagram of an ultrasonic diagnostic apparatus 1 related to the present embodiment of the present invention.

The ultrasonic diagnostic apparatus 1 is configured by transmission means 11, transmission/reception separating means 12, a probe 13, reception means 14, phasing addition means 15, ultrasonic tomographic image calculation means 16, signal analysis means 17, black and white information converting means 18, switching/adding means 19, display means 20, displacement amount calculation means 21, elasticity calculation means 22, elasticity data analysis means 23, color signal data converting means 24, console 25, and ROI data calculation means 26, etc.

The transmission means 11 generates ultrasonic waves for transmitting to an object 3, and the transmission/reception separating means 12 switches for transmission or reception of ultrasonic waves. The probe 13 is to be applied to the object 3 for transmitting/receiving ultrasonic waves to/from the object, and the reception means 14 amplifies the ultrasonic echo signals with respect to the ultrasonic waves transmitted from the probe 13. The phasing addition means 15 performs predetermined delay process with respect to the ultrasonic echo signals received by the reception means 14, and performs phasing addition.

The ultrasonic tomographic image calculation means 16 calculates ultrasonic tomography data of biological tissues based on the output signals from the phasing addition means 15. The signal analysis means 17 calculates the representative value of an ultrasonic tomographic image (for example, the average value of pixel value of an arbitrary region) from the output signals of the ultrasonic tomographic image calculation means 16. The black and white data converting means 18 generates ultrasonic tomography data from the output signals of the ultrasonic tomographic image calculation means 16. The display means 20 displays an ultrasonic tomographic image based on the tomography data.

The ROI data calculation means 26 is for performing calculation process related to the divided regions by extracting positional information of ROI or included beam number etc. displayed on the display means 20, and the console 25 is for inputting numerical values for setting or commands, etc. related to division of ROI.

The displacement calculation means 21 calculates displacement amount between the frames of biological tissues included in an ROI displayed on the display means 20 based on the signals outputted from the phasing addition means 15. The elasticity calculation means 22 calculates elasticity data such as strain or elasticity modulus of biological tissues in an ROI from the displacement amount calculated by the displacement amount calculation means 21. The elasticity data analysis means 23 calculates the representative value of elasticity data calculated by the elasticity calculation means 22.

The color signal information converting means 24 generates color signal information for displaying the output signals of the elasticity data analysis means 23 as a color image on the display means 20, and the switching/adding means 19 switches the various display methods such as adding the output signals of the black and white information converting means 18 and the color signal information converting means 24 to display on the display means 20.

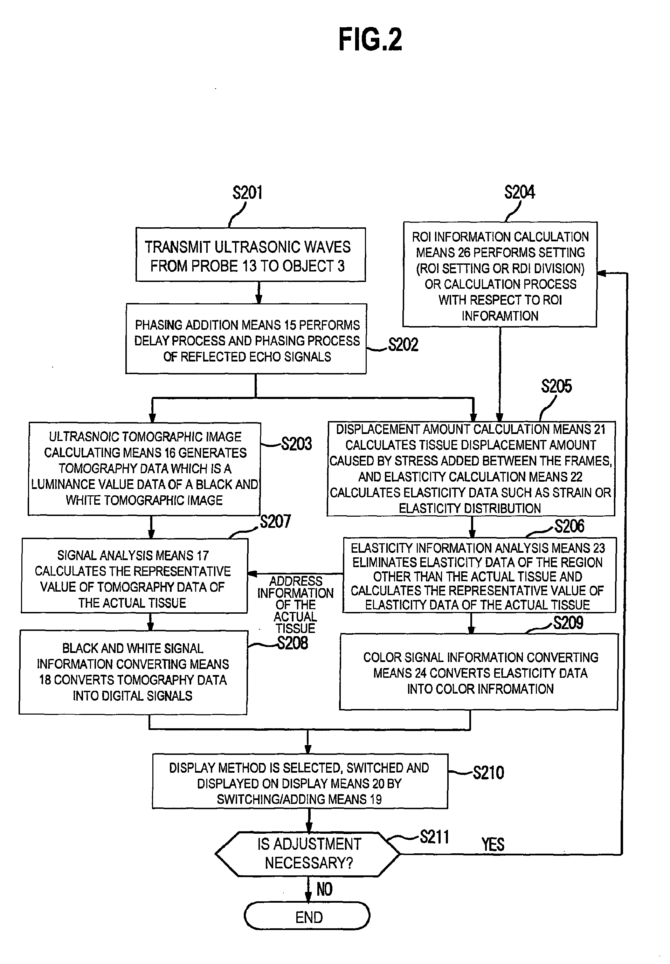

Next, diagnostic process to be performed by the ultrasonic diagnostic apparatus 1 will be described. Fig. 2 is a general flow chart indicating processing procedure to be performed by the ultrasonic diagnostic apparatus 1.

The transmission/reception separating means 12 transmits ultrasonic waves from the probe 13 to the object 3 (step 201), receives the ultrasonic echo signals, and the phasing addition means 15 performs process such as delay process and phasing process on the ultrasonic echo signals (step 202).

The ultrasonic tomographic image calculation means 16 generates tomography data that is luminance value data of a black and white tomographic image (step 203).

Also, the ROI data calculation means 26 performs the setting related to ROI data (ROI setting or ROI division) or calculation process (step 204), the displacement amount calculation means 21, in the set ROI, calculates displacement amount of biological tissues caused by the stress added between the frames based on the output from the phasing addition means 15, and the elasticity calculation means 22 calculates elasticity data such as strain or elasticity distribution of biological tissues from the calculated tissue displacement amount (step 205).

Next, the elasticity data analysis means 23 eliminates elasticity data of the region other than actual tissues, and calculates the representative value of elasticity data of the actual tissues (step 206).

The signal analysis means 17 receives address information of actual tissues from the elasticity data analysis means 23 and calculates the representative value of the tomography data of the actual tissues (step 207).

The black and white information converting means 18 converts tomography data into digital signals (step 208), and the color signal information converting means 24 converts elasticity data into color information (step 209). The switching/adding means 19 selects and switches display methods, and displays tomography data, elasticity data or an overlapped data of the two (Step 210).

The operator diagnoses the elasticity data and tomography data displayed on the display means 20, determines whether adjustment such as setting of ROI or recalculation (step 211), returns to the step 204 if necessary and performs the setting or calculation process related to the ROI data and recalculates elasticity data in the set ROI.

Next, each step will be described in detail.

### (step 201, step 202)

The transmission/reception separating means 12 transmits the ultrasonic waves transmitted from the transmission means 11 to the object 3 via the probe 13.

The transmission means 11 has a function to generate transmission pulses for generating ultrasonic waves by driving the probe 13 and to set the convergent point of the transmitted ultrasonic waves at a certain depth.

The transmission/reception separating means 12 is configured by a switching circuit, and performs switching of communication channels by transmitting transmission pulse generated by the transmission means 11 to the probe 13 upon transmission of ultrasonic waves and transmitting the ultrasonic echo signals from the object 3 to the reception means 14 upon reception of ultrasonic waves.

The probe 13 is formed by arranging a plurality of transducers, and has a function for performing beam scanning electronically to transmit/receive ultrasonic waves to/from the object 3 via the transducers.

The reception means 14 is for amplifying the ultrasonic echo signals received by the probe 13 at a predetermined gain so as to generate ultrasonic reception signals.

The phasing addition means 15 performs phase control after inputting ultrasonic reception signals amplified in the reception means 14.

### (Step 203)

The ultrasonic tomographic image calculation means 16 performs a variety of signal processing such as gain compensation, compression, detection, edge enhancement and filtering with respect to the ultrasonic reception signals outputted from the phasing addition means 15, and generates tomography data that is luminance value data of an ultrasonic black and white tomographic image.

On the basis of such tomography data, a tomographic image is displayed on the display means 20 in real time.

### (Step 204)

Next, a process related to set an ROI or divide the ROI thereof by the ROI data calculation means 26 will be described.

Setting of ROI, etc. is carried out by displaying tomographic images, etc on the display means 20 and inputting an ROI range or setting value, etc. by the console 25.

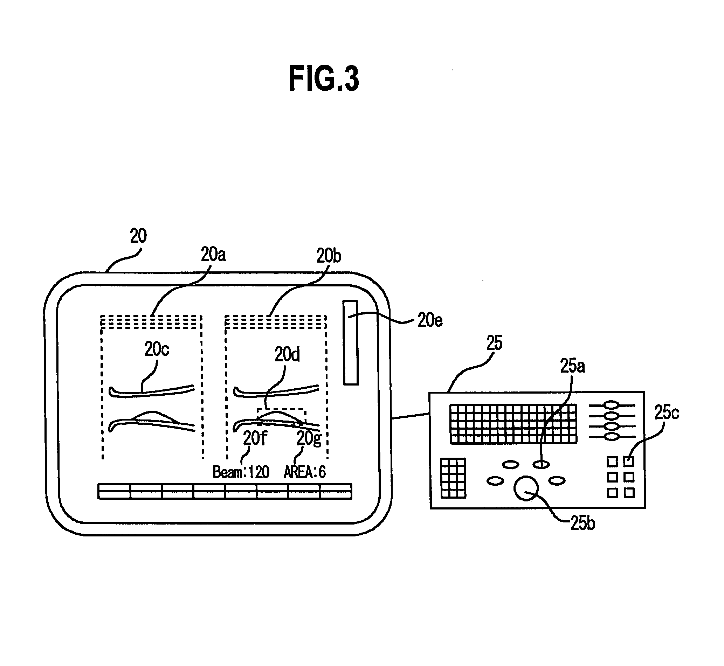

Fig. 3 shows an example of screens to be displayed on the display means 20 and the console 25. On the display screen 20, for example, a tomographic image screen 20a showing the tomographic image displayed based on the tomography data generated by the ultrasonic tomographic imaging calculation means 16 and an elastic image screen 20b showing elasticity data being superimposed over the tomographic image.

A biological tissue 20c being a diagnosis target is displayed on the tomographic image screen 20a and the elastic image screen 20b, and an ROI (Region Of Interest) 20d for calculating elasticity data is displayed on the elastic image screen 20b. A color bar 20e is for indicating the calculated value of elasticity data or range of the value, etc. by colors.

Here, an embodiment will be described by exemplifying a plaque accumulated in an arterial vessel as a biological tissue 20c.

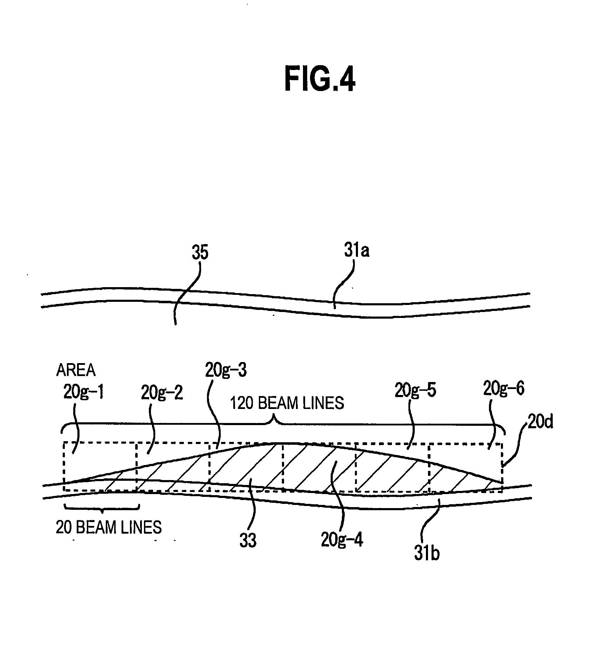

Fig. 4 is an enlarged view of the vicinity of ROI 20d displayed on the elastic image screen 20b.

By pushing an elasticity mode transition button 25a on the console 25, the display screen of the ultrasonic diagnostic apparatus 1 is switched from the screen shown in Fig. 3 to the elastic image display screen shown in Fig. 4. The operator, as shown in Fig. 4, sets an ROI 20d with respect to the plaque accumulated in the arterial vessel using the trackball 25b, etc. in the console 25.

In Fig. 4, the upper vessel wall and the lower vessel wall are indicated by 31a and 31b, and the blood flows in the vessel is indicated by 35. By the ultrasonic diagnostic apparatus 1, it is diagnosed that the plaque 33 is accumulated at the lower Bessel wall 31b from the tomographic image 20a. Accordingly, the operator sets the ROI 20d via the console 25 for calculating and displaying elasticity data of the plaque 33.

With respect to the set ROI 20d, the ROI data calculation means 26 performs counting of positional information of the ROI 20d, the number of ultrasonic beams, data points included in the depth direction, etc. included in the ROI 20d, stores the counted values and displays the counted number of beam lines on a beam number display unit 20f. In this case, as shown in Fig. 3, "120" is displayed on the beam number display unit 20f, and 120 ultrasonic beams are transmitted/received to/from the ROI 20d.

Also, the operator specifies the number of divided regions of the ROI 20d using an operation input button 25c, etc. Here, the divided regions means that the ROI is divided into a plurality of regions, and elasticity data can be calculated and displayed for the divided regions. For example, when it is inputted from the console 25 to divide the ROI 20d into six regions, the number of divided region is displayed as "6" in a divided region number display unit 20g on the display screen shown in Fig. 3.

When the number of divided regions is set as "6", the ROI data calculation means 26 divides the ROI 20d into 6 regions of areas 20g-1, 20g-2, 20g-3, 20g-4, 20g-5 and 20g-6, and displays them on the display means 20 as shown in Fig. 4. Here, the number of ultrasonic beams included in one divided region is 20.

In this way, the ROI data calculation means 26 performs the setting related to ROI setting or region dividing, and keeps the ROI data related to the settings thereof.

### (Step 205 and step 206)

Next, calculation of elasticity data will be described.

By imaging an arterial vessel of the object 3 using the probe 13, ultrasonic echo signals for calculating elasticity data is inputted to the displacement amount calculation means 21. The displacement amount calculation means 21 calculates the displacement amount of biological tissues between the predetermined time frames.

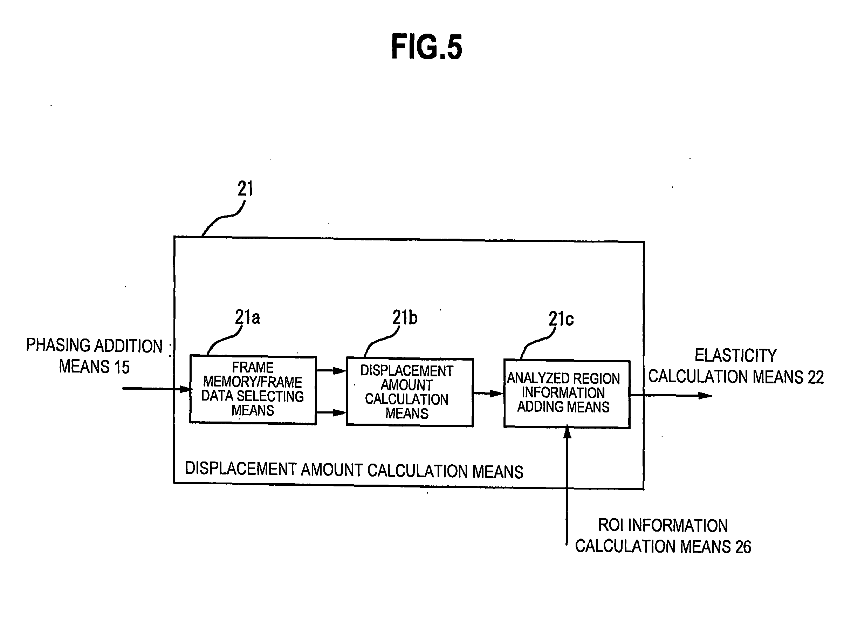

Fig. 5 is a block configuration diagram of the displacement amount calculation means 21. The displacement amount calculation means 21 is configured by a frame memory/frame data selecting means 21a, the displacement amount calculation means 21b and an analyzed region information adding means 21c. Ultrasonic echo signals (by frame units) are sequentially inputted in real time from the phasing addition means 15, and the ultrasonic echo signals for the portion of plurality of frames are stored to the frame memory/frame data selecting means 21a.

From the ultrasonic echo signals of the stored frame unit, frame data for the portion of two frames to which an adequate stress is added is selected, and outputted to the displacement amount calculation means 21b. That is, to the displacement amount calculation means 21b, ultrasonic echo signals of two frame units are generally inputted.

Next, the displacement amount calculation means 21b calculates displacement amount of the tissues included in the ROI 20d being set on the display means 20 from the ultrasonic echo signals of two frame units that are the output signals from the frame memory/frame data selecting means 21a using, for example, the block matching method.

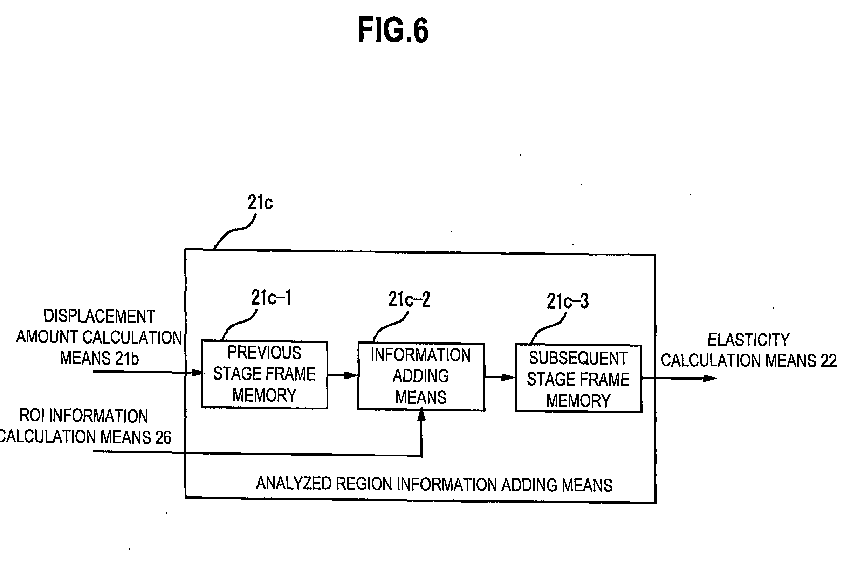

Fig. 6 is a block configuration diagram of the analyzed region information adding means 21c. The analyzed region information adding means 21c is configured by a former step frame memory 21c-1, an information adding unit 21c-2 and a latter step frame memory 21c-3. The former step frame memory 21c-1 is for storing data of a frame unit outputted from the displacement amount calculation means 21b. The information adding means 21c-2 is for adding a region identification data 43 for identifying divided regions (for example, the coordinate of the border of a divided region) with respect to the data stored in the former step frame memory 21c-1 based on the ROI information stored in the ROI information calculation means 26.

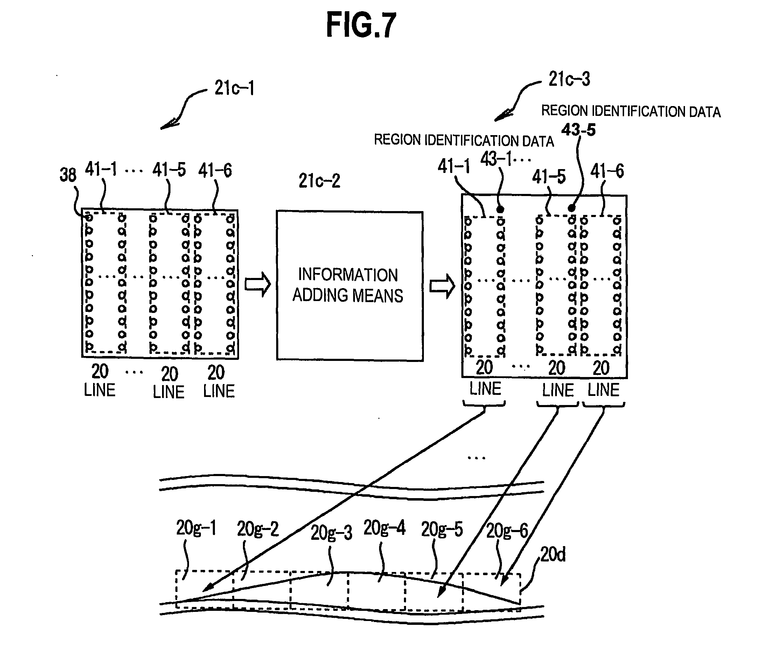

Fig. 7 illustrates the addition of the region identification data 43. In the former step frame memory 21c-1, a displacement amount data 38 of the tissue in each point is sequentially stored in each divided region of the ROI 20d. The displacement data 38 is the displacement amount calculated in the respective pixels of the tomographic image.

To the information adding means 21c-2, the number of ultrasonic beams included in the respective areas 20g-1, ...1, 20g-6 of the divided region (for example, "20" lines), address information of the starting point of the ROI 20d, the value of the data points in the depth direction, etc. are inputted from the ROI data calculation means 26. On the basis of the values thereof, the information adding means 21c-2 adds the region identification data 43-1, ..., 43-5 for identifying the border of the divided regions 20g-1, ..., 20g-6 with respect to the displacement amount data 38 stored in the former step frame memory 11c-1, and stores them in the latter frame memory 21c-3.

For example, when the block of the displacement amount data 38 corresponding to the area 20g-1 is set as 41-1, the block of the displacement amount data 38 corresponding to the area 20g-5 is set as 41-5 and the block of the displacement amount data 38 corresponding to the area 20g-6 is set as 41-6, the information adding means 21c-2 adds a region identification data 43-1 between the block 41-1 and the block 41-2, ..., and region identification data 43-5 between the block 41-5 and the block 41-6.

In this way, the displacement amount calculation means 21 of the present ultrasonic diagnostic apparatus 1 calculates the displacement amount data to which the region identification data 43 for identifying for each divided region 20g is added, and outputs the calculated data to the elasticity calculation means 22.

In this manner, the ultrasonic diagnostic apparatus 1 is capable of identifying the border portion of the respective divided regions 20g in common in each signal processing by the region identification data 43.

When the divided region 20g is not set in the ROI 20d, the displacement amount is outputted to the elasticity calculation means 22 without adding the region identification data 43.

The elasticity calculation means 22 calculates elasticity data such as strain or the Young's modulus based on the displacement amount between the frames calculated in the displacement amount calculation means 21.

The elasticity calculation means 22 calculates strain by performing spatial differentiation process with respect to the displacement amount between the frames calculated by the displacement amount calculation means 21. By performing this process for one frame, it is possible to calculate strain distribution.

Also, the elasticity calculation means 22 sets the stress to be added between the time phases "n" and "n+1" as ΔP and the strain generated upon the addition of the stress thereof as Δ ε, and calculates the Young's modulus by Y=ΔP/Δε. By performing this process for one frame, it is possible to obtain elasticity modulus distribution.

Such calculated data is outputted to the elasticity data analysis means 23.

The elasticity data analysis means 23 eliminates elasticity data other than the elasticity data of actual tissues from among the elasticity data calculated by the elasticity calculation means 22, calculates the representative value of elasticity data of the actual tissues, and outputs address information of the actual tissues to the signal analysis means 17.

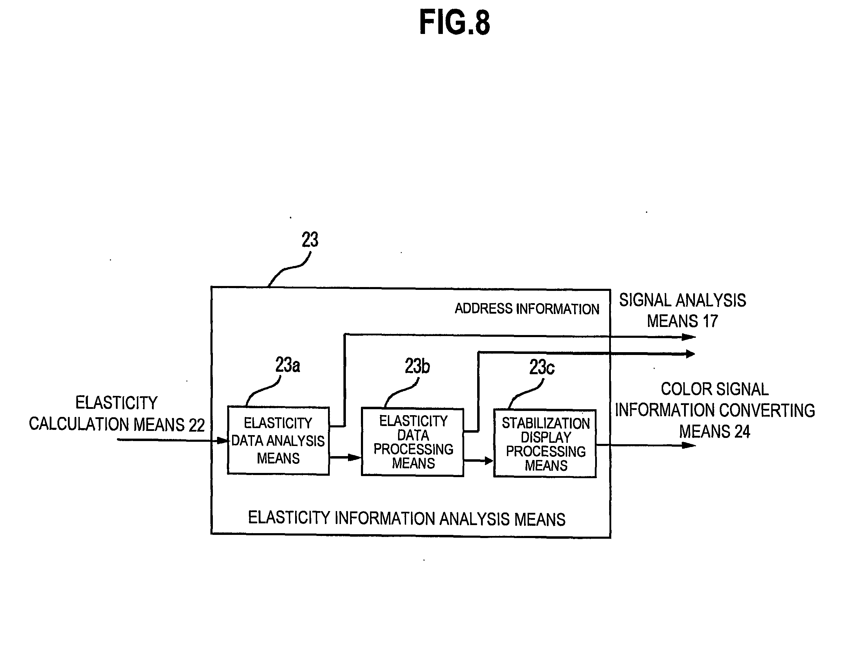

Fig. 8 is a block configuration diagram of the elasticity data analysis means 23. The elasticity data analysis means 23 is configured by an elasticity data analysis means 23a, an elasticity data processing means 23b and a stabilization display processing means 23c.

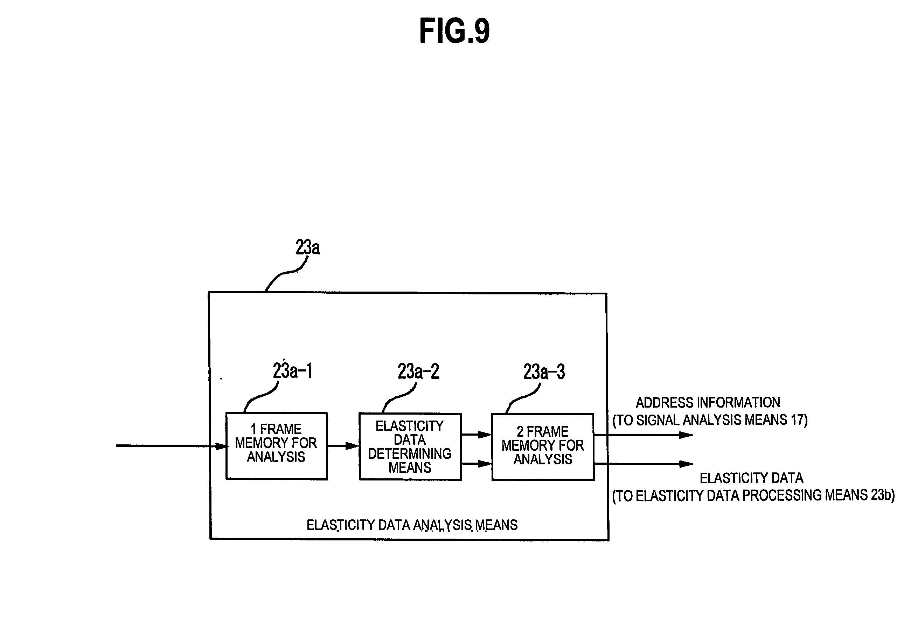

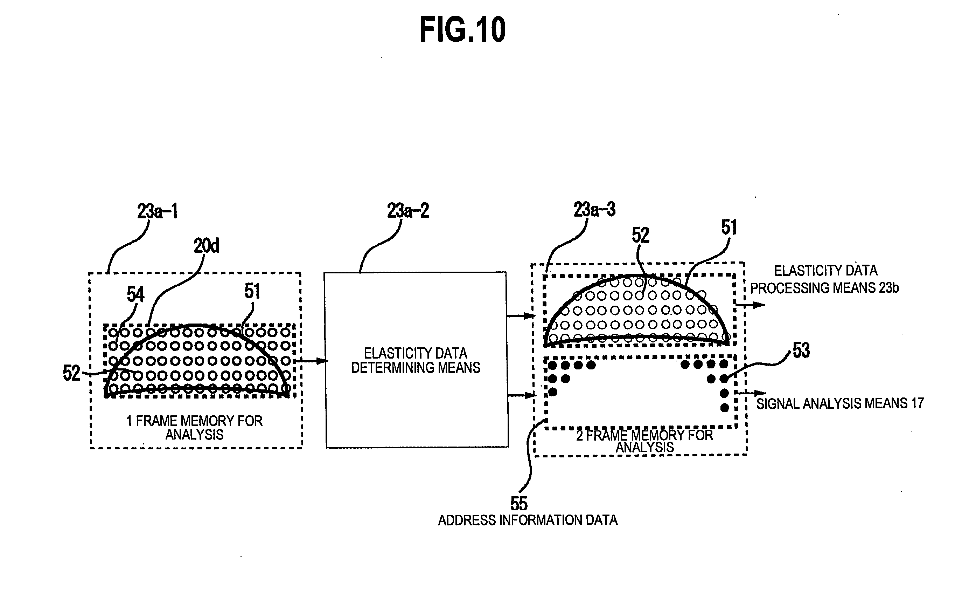

Fig. 9 is a block configuration diagram of the elasticity data analysis means 23a, and Fig. 10 illustrates the process to be performed by the elasticity data analysis means 23a.

The elasticity data analysis means 23a is configured by a one-frame memory 23a-1 for analysis, elasticity data determining means 23a-2 and two-frame memory 23a-3 for analysis.

As shown in Fig. 10, elasticity data in the ROI 20d such as strain or the Young's modulus is stored in the one-frame memory 23a-1 for analysis. In the ROI 20d, there is a part of the actual tissue 51 and an elasticity data 52 corresponding to the pixel included in strain included therein, and a part of the region 53 which is a region other than the actual tissue and an elasticity data 54 corresponding to the pixel included therein.

Here, the actual tissue 51 is a plaque 33 accumulated in an arterial vessel, and the region 53 which is the region other than the actual tissue is the region of blood 35 that flows in the arterial vessel. Generally, elasticity data of the region other than actual tissues has a tendency of fluctuating. Therefore, in order to obtain the representative value of elasticity data with accuracy, it is necessary to obtain the representative value of the elasticity data 52 of the actual tissue 51.

For that reason, the elasticity data determining means 23a-2 of the elasticity data analysis means 23a differentiates elasticity data 52 of the actual tissue 51 and elasticity data 54 of the region 53 which a region other than the actual tissue 51 from elasticity data of the ROI 20d stored in the one-frame memory 23a-1 for analysis based on, for example, the degree of temporal and spatial dispersion of the elasticity data, abstracts the elasticity data 52 of the actual tissue 51 and address information data 55 of the pixel included in the region 53 which is a region other than the actual tissue 51, and stores the abstracted data to the two-frame memory 23a-3 for analysis.

The elasticity data 52 stored in the two-frame memory 23a-3 for analysis from which the noise component is eliminated is outputted to the elasticity data processing means 23b of the latter step, and the address information data 55 is outputted to the signal analysis means 17 to be described later.

In this manner, elasticity data of the actual tissue can be accurately calculated for each divided region.

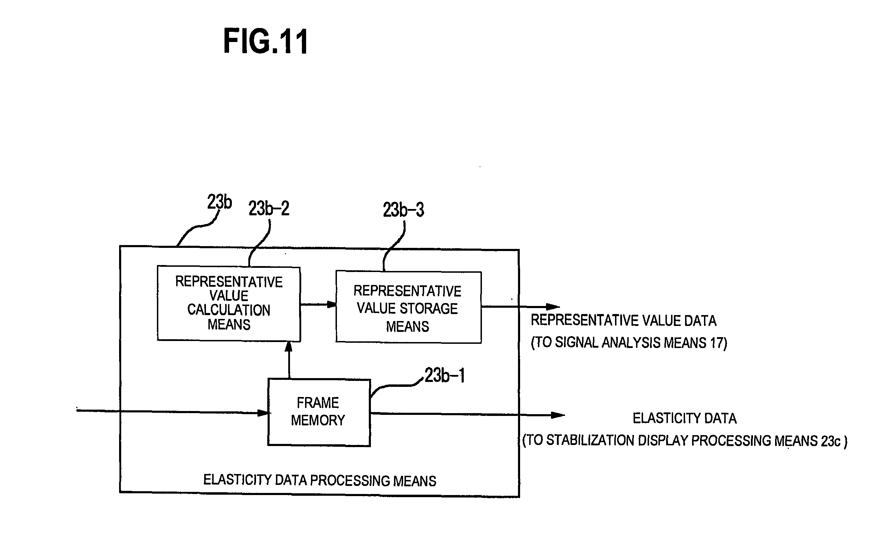

Fig. 11 is a block configuration diagram of the elasticity data analysis means 23b. The elasticity data analysis means 23b is configured by a frame memory 23b-1, a representative value calculation means 23b-2 and a representative value storage means 23b-3. The elasticity data processing means 23b stores the received elasticity data 52 to the frame memory 23b-1, and the representative value calculation means 23b-2 performs statistical processing on the elasticity data 52 and calculates the representative value thereof (for example, the average value, etc.).

The representative value storage means 23b-3 stores the representative value calculated by the representative value calculation means 23b-2. As shown in Fig. 4, in the case that the ROI 20d is divided into six regions of the divided regions 20g-1, ..., 20g-6, the representative value calculation means 23b-2 calculates the representative value in the respective divided regions 20g, and stores the respective representative values to the representative value storage means 23b-3.

In this way, the representative value stored in the representative value storage means 23b-3 is outputted to the signal analysis means 17.

Also, the elasticity data 52 stored in the frame memory 23b-1 is outputted to the stabilization display processing means 23c.

### (Step 207 ∼ step 209)

The signal analysis means 17 receives the address information data 55 of the region other than the actual tissue 51 from the elasticity information analysis means 23, and calculates the representative value of tomography data of the actual tissue 51.

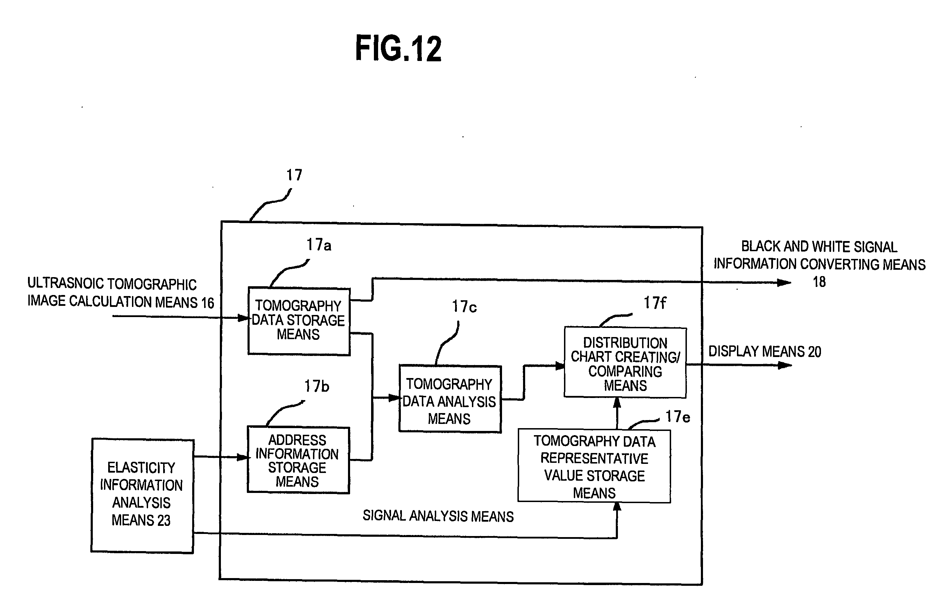

Fig. 12 is a block configuration diagram of the signal analysis means 17. The signal analysis means 17 is configured by a tomography data storage means 17a, an address information storage means 17b, a tomography data analysis means 17c, an elasticity data representative value storage means 17e and a distribution chart creating/comparing means 17f.

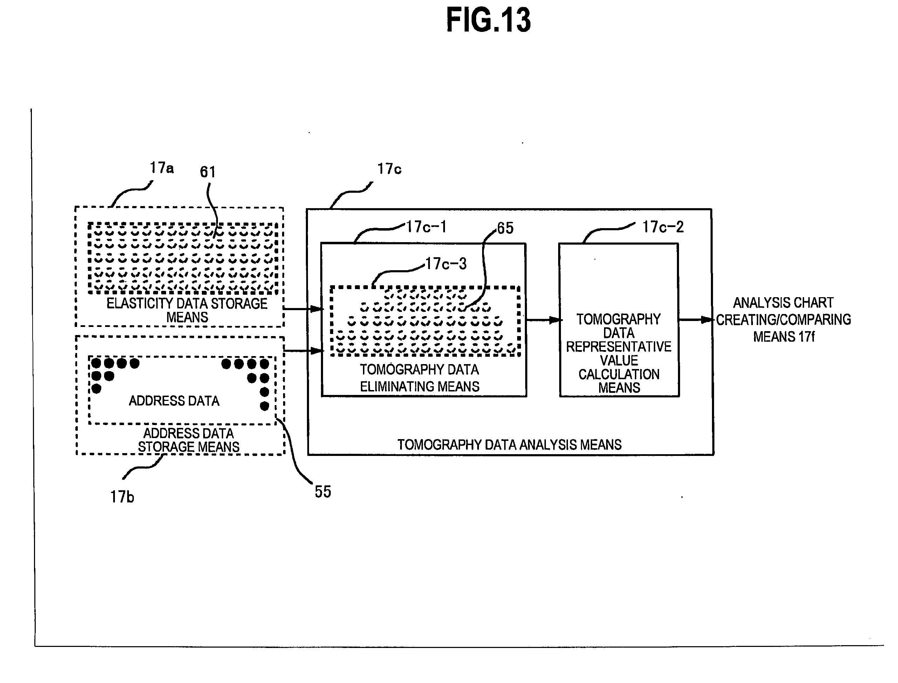

Fig. 13 is an explanatory diagram of the process for calculating the representative value of tomography data.

The tomography data storage means 17a is for temporarily storing tomography data of the ultrasonic tomographic image calculated by the ultrasonic tomographic image calculation means 16 in real time, and outputting the imaged data in real time to the black and white signal data converting means 18 of the latter step.

The address information storage means 17b stores the address information data 55 outputted from the elasticity data analysis means 23.

The tomography data analysis means 17c is configured by a tomography data eliminating means 17c-1 and a tomography data representative value calculation means 17c-2 as shown in Fig. 13. The tomography data eliminating means 17c-1 eliminates the data corresponding to the region 53 which is a region other than the actual tissue 51 from the tomography data 61 stored in the tomography data storage means based on the address information data 55 stored in the address information storage means 17b, and extracts the tomography data 65 corresponding to the actual tissue 51.

In this manner, it is possible to accurately calculate tomography data of actual tissues for each divided region.

The tomography data representative value calculation means 17c-2 performs statistical processing on the tomography data (luminance value data) 65 corresponding to the actual tissue 51, and calculates the representative value (average value, etc.) with respect to the luminance value. The calculated representative value of the tomography data 65 is outputted to the distribution chart creating/comparing means 17f of the latter step.

Representative value of the elasticity data outputted from the elasticity data analysis means 23 is stored in the elasticity data representative value storage means 17e, and it is to be outputted to the distribution chart creating/ comparing means 17f.

The distribution chart creating/comparing means 17f develops a distribution chart based on the inputted representative value of the tomography data and the representative value of the elasticity data, and outputs the distribution chart indicating the relationship of the both values, etc. to the display means 20.

The black and white information converting means 18 is what is called a scan converter, and is configured including an A/D converter for converting tomography data for ultrasonic black and white tomographic images stored in the tomography data storage means 17a of the signal analysis means 17, a frame memory for storing the plurality of converted tomography data in time series, and a controller. It is for obtaining the tomography frame data in the object 3 stored in the frame memory of the black and white information converting means 18 as one image and reading out the obtained tomography frame data in TV synchronism, and the read out data is displayed on the display means 20 via the switching/adding means 19.

Next, display of elasticity data will be described.

The stabilization display process means 23c of the elasticity data analysis means 23, in order to display the elasticity data 52 stored in the frame memory 23b-1 in the elasticity data processing means 23b as a stabilized image to the display means 20, for example, in the case that the calculated strain is extremely small, eliminates the frame thereof and performs signal processing such as smoothing process by the previous and subsequent frames in time direction.

Then the signal processed elasticity data 52 is converted into color codes by the color signal data converting means 24. The color signal data converting means 24 converts the data into 3 primary colors of light which are red(R), green(G) and blue (B) based on the elasticity data 52. For example, elasticity data with large strain is converted into red code and elasticity data with small strain is converted into blue code. The gradient of red(R), green(G) and blue(B) has 256 shades.

### (Step 210)

Tomography data or the representative value thereof, elasticity data or the representative value thereof, etc. calculated as above are displayed to the display means 20 by a variety of methods specified and switched by the switching/adding means 19.

Hereinafter, several display examples will be described.

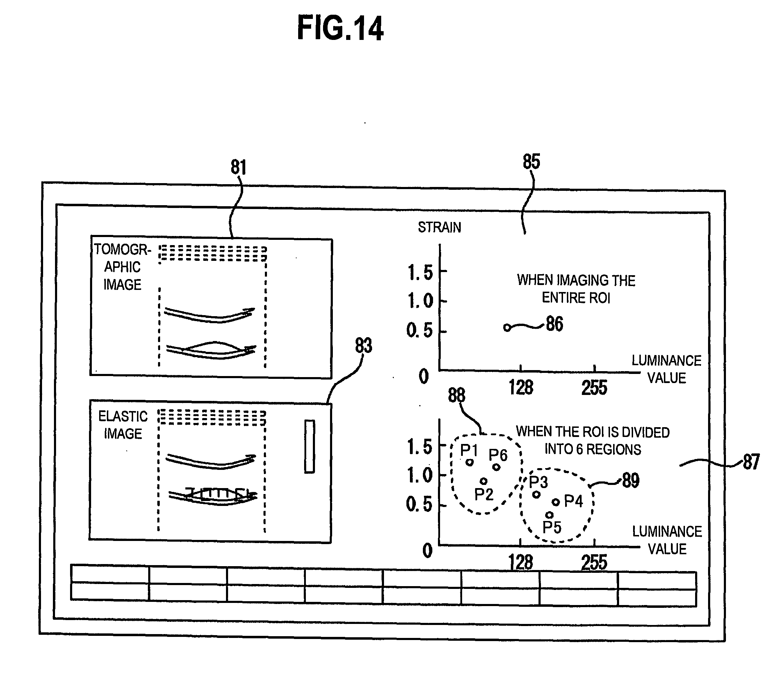

Fig. 14 is a display example showing the distribution chart of the representative value of tomography data and elasticity data. On the screen of the display means 20, a tomographic image 81, elasticity image 83, distribution chart 85 and distribution chart 87 are displayed.

The tomographic image 81 is the image displayed based on the tomography data stored in the tomography data storage means 17a of the signal analysis means 17.

The elasticity image 83 is the image displayed by superimposing over the tomographic image 81 based on the elasticity data 52 colored by the color signal data converting means 24.

The lateral axis of the distribution chart 85 and the and the distribution chart 87 represents, for example, luminance value of the tomography data in the tissues included in the ROI 20d, and the vertical axis represents strain of the tomography data in the tissues included in the ROI 20d.

The distribution chart 85 shows a point 86 indicating the relationship between the representative value of the luminance value that is tomography data in the entire region of ROI 20d and the representative value of elasticity data.

The distribution chart 87 shows points P1, P2, P3, P4, P5 and P6 indicating the relationship between the representative value of the luminance value that is the tomography data and the representative value of the elasticity data in each divided region in the case that the ROI 20d is divided into six regions of the divided regions 20g-1, ..., and 20g-6. From the distribution chart 87, it can be recognized that the points P1, P2 and P6 included in a group 88 have lower luminance value and larger strain, and the points P3, P4 and P5 included in a group 89 have higher luminance and smaller strain.

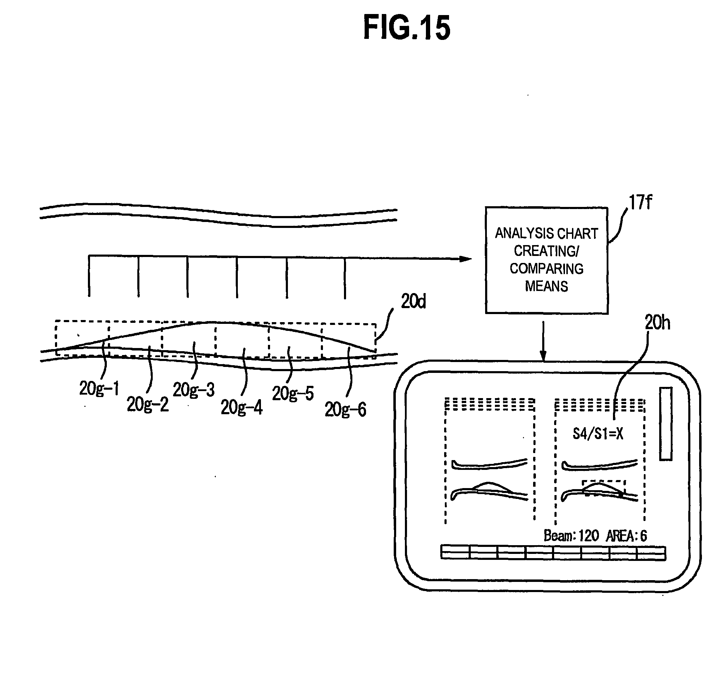

Fig. 15 shows an operation and display example by the distribution chart creating/comparing means 17f.

As shown in Fig. 15, in the case that the ROI 20d is divided into six regions of the divided regions 20g-1, ..., and 20g-6, the representative values of elasticity data in the respective regions are set as S1, ..., and S6. These representative values S1, ..., and S6 are stored in the distribution chart creating/comparing means 17f, and if the comparison of the representative values is commanded, they can be displayed as a comparison result 20h. In this way, by comparing the representative values, it is possible to evaluate hardness of tissues, etc. for each divided region quantitatively. In this regard, however, the object of comparison is not limited to the representative values.

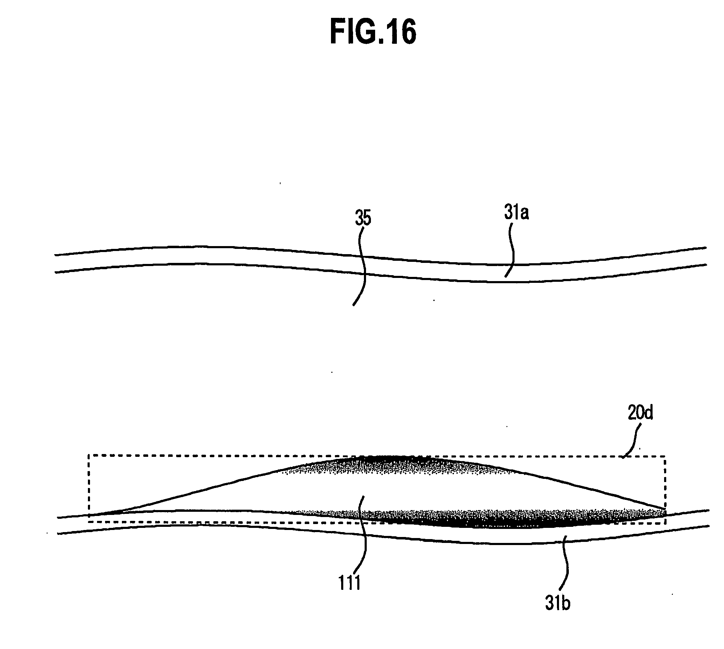

Fig. 16, Fig. 17 and Fig. 18 show the display examples of elasticity data distribution.

The present ultrasonic diagnostic apparatus 1, as a display method of elasticity images, is capable of translucently displaying an elastic image over a tomographic image, and specifying a hue such as color display or gray scale.

Fig. 16 is a display example of an elastic image 111 indicating distribution of the elasticity data in the ROI 20d in the case that the entire ROI 20d is specified.

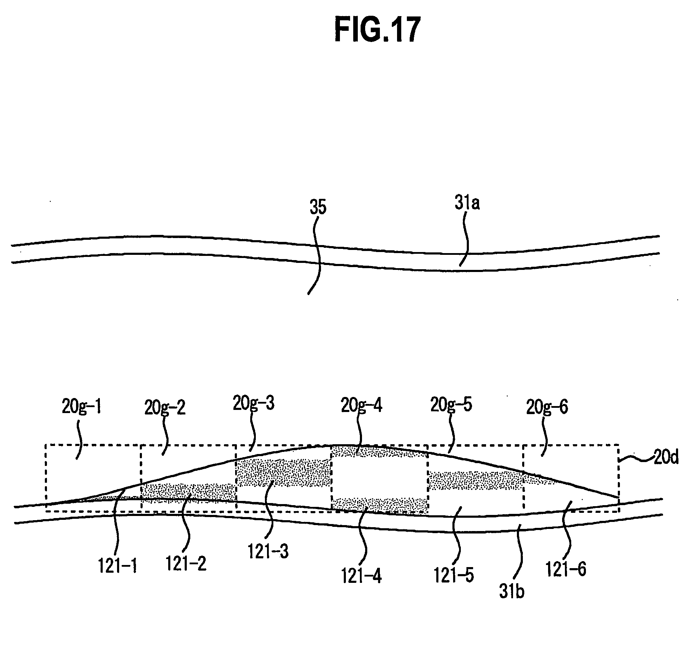

Also, Fig. 17 is a display example of elastic images 121-1, 121-2, 121-3, 121-4, 121-5 and 121-6 indicating distribution of elasticity data in the case that the ROI 20d is divided into six regions of the divided regions 20g-1, 20g-2, 20g-3, 20g-4, 20g-5 and 20g-6.

The ultrasonic diagnostic apparatus 1, in the respective divided regions 20g-1, ..., 20g-6, is capable of displaying an elastic image indicating distribution of elasticity data for each divided region by specifying the maximum value and the minimum value of the elasticity data, as shown in the display example of Fig. 17.

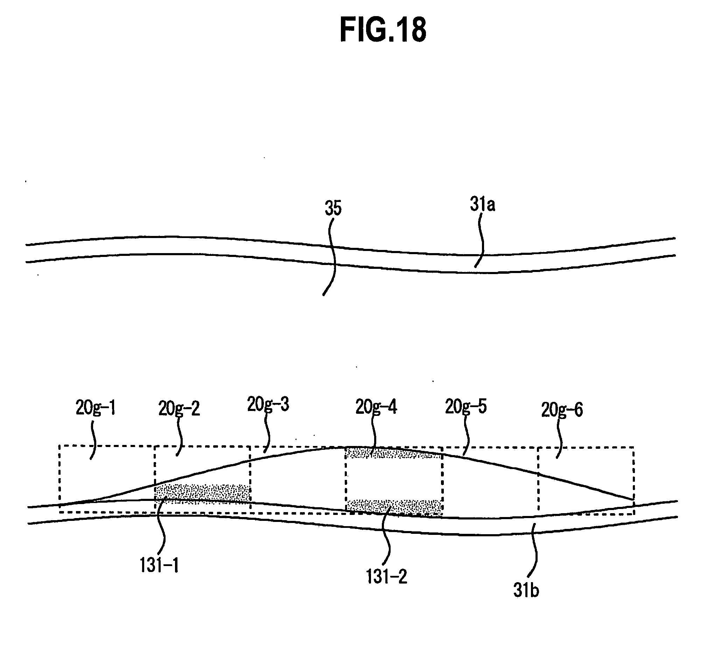

Also, the ultrasonic diagnostic apparatus 1 can display an elastic image of the specified divided region. For example, as shown in the display example of Fig. 18, by specifying the divided regions 20g-2 and 20g-4, elastic images 131-1 and 131-2 of the divided regions can be displayed.

As described above, the ultrasonic diagnostic apparatus 1 is capable of handling the region of interest by dividing it into a plurality of regions for analysis. Also, the analysis result can be displayed for each divided region. The ultrasonic diagnostic apparatus can also display the calculated tomography data or the representative value thereof and elasticity data or the representative value thereof, etc., and display them using a variety of display methods, whereby making it possible to easily review and examine the display for diagnosis.

In this manner, on the basis of the display result on the display means 20, the ROI can be set again if necessary, and the calculation can be performed again by repeating the process from the step 204 to the step 210 (step 211).

While the example of the ultrasonic probe 13 of linear electronic scanning-type wherein the ROI 20d is a rectangle is described in the present embodiment, it is also applicable to the probe to which an other scanning method is adopted.

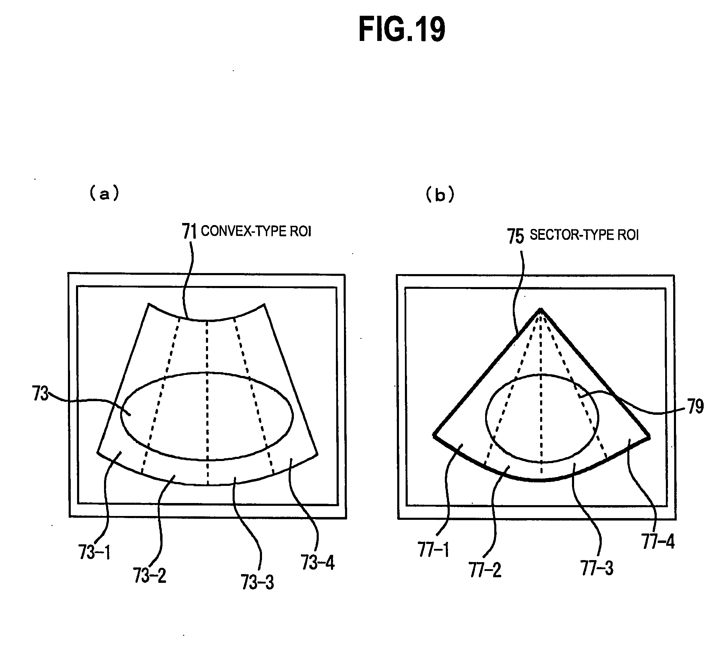

Fig. 19 shows the setting method example of the kind of the probe 13 and the ROI.

Fig. 19(a) shows an ROI 73 of the convex-type probe. The convex-type ROI 73 including a diagnostic target tissue 73 is divided into four divided regions 73-1, 73-2, 73-3 and 73-4, and can perform the same analysis as the previously described analysis.

Fig. 19(b) shows an ROI 75 of the sector-type probe. The sector-type probe ROI 75 including a diagnostic target tissue 79 is divided, for example, into four divisional regions 77-1, 77-2, 77-3 and 77-4, and the same analysis can be performed as the previously described one.

In this way, in accordance with the present ultrasonic diagnostic apparatus, it is possible to accurately calculate elasticity information of target tissues, and to perform ultrasonic diagnosis from multiple aspects by visualizing the target tissues using a variety of display methods.

While preferable embodiments of the ultrasonic diagnostic apparatus related to the present invention are described above referring to the attached diagrams, no limitations are intended to the above-described examples. It is therefore evident for those skilled in the art that the particular embodiments disclosed above may be altered or modified and all such variations are considered within the scope and spirit of the invention.

## Claims

1. An ultrasonic diagnostic apparatus comprising:
an ultrasonic probe for transmitting/receiving ultrasonic waves to/from an object to be examined;
elasticity data calculation means for calculating elasticity data of tissues based on the output signals from the ultrasonic probe;
tomography data calculation means for calculating tomography data indicating form of the tissues based on the output signals from the ultrasonic probe; and
display means for displaying the elasticity data and the tomography data,
**characterized in** further comprising:
ROI (region of interest) dividing/setting means for setting an ROI formed by a plurality of divided regions,
wherein calculation of elasticity data by the elasticity data calculation means and/or calculation of tomography data by the tomography data calculation means are performed for the divided regions.

2. The ultrasonic diagnostic apparatus according to claim 1,
wherein the display means displays the elasticity data and the tomography data for the divided regions.

3. The ultrasonic diagnostic apparatus according to claim 1,
wherein the elasticity calculation means comprises elasticity data analysis means for calculating the representative value of elasticity data for the divided regions.

4. The ultrasonic diagnostic apparatus according to claim 3,
wherein the tomography data calculation means comprises signal analysis means for calculating the representative value of tomography data for the divided regions.

5. The ultrasonic diagnostic apparatus according to claim 4,
wherein the signal analysis means comprises distribution chart creating/comparing means for creating a distribution chart of the tomography data representative value and the elasticity data representative value, and outputting the created distribution chart to the display means.

6. The ultrasonic diagnostic apparatus according to claim 5,
wherein the distribution chart creating/comparing means creates a distribution chart for the entire region of interest or for the divided regions.

7. The ultrasonic diagnostic apparatus according to claim 3,
wherein the display means displays the ratio of the representative value of the elasticity data calculated for the divided regions.

8. The ultrasonic diagnostic apparatus according to claim 3,
wherein the elasticity data analysis means comprises:
elasticity data analysis means for differentiating a region of actual tissues and a region other than the actual tissues based on elasticity data, and outputting address information of both regions; and
elasticity data processing means for calculating the representative values of elasticity data of an actual tissue region and the region other than the actual tissues.

9. The ultrasonic diagnostic apparatus according to claim 8,
wherein the elasticity data analysis means comprises elasticity data determining means for differentiating an actual tissue region and a region other than the actual tissues based on dispersion of elasticity data.

10. The ultrasonic diagnostic apparatus according to claim 8,
**characterized in** comprising means for performing calculation by eliminating elasticity data and tomography data of the region other than actual tissues based on the address information.

11. The ultrasonic diagnostic apparatus according to claim 1,
wherein the elasticity data calculation means comprises analyzed region information adding means for adding region identification data that identifies boundary of the divided regions to the elasticity data.
